# EUROPEAN PATENT SPECIFICATION

(11) **EP 4 029 515 B1**
(45) Date of publication and mention of the grant of the patent: **04.12.2024**
(21) Application number: 19938050.2
(22) Date of filing: 17.07.2019
(51) Int. Cl.: A61K 31/711, A61K 38/17, C12Q 1/6883, A61K 49/00, A61P 31/00, A61K 45/00

(54) **ANTI-INFECTION EFFECTS OF HNRNPA2B1 AND USE THEREOF**
ANTIINFEKTIONSUNGSWIRKUNG VON HNRNPA2B1 UND DEREN VERWENDUNG
EFFETS ANTI-INFECTIEUX DE LA HNRNPA2B1, ET UTILISATION DE CELLE-CI

(43) Date of publication of application: 20.07.2022
(73) Proprietor: Institute of Basic Medical Sciences, Chinese Academy of Medical Sciences, Beijing 100730 (CN)
(72) Inventor: CAO, Xuetao, Beijing 100730 (CN); WANG, Lei, Shanghai 200433 (CN); WEN, Mingyue, Shanghai 200433 (CN)
(74) Representative: EP&C
(86) International application number: PCT/CN2019/096303
(87) International publication number: WO 2021/007800

(56) References cited:
- WO-A1-2005/059138
- WO-A1-2018/215400
- CN-A- 102 887 950
- CN-A- 104 388 428
- US-A1- 2003 068 803
- KATOH HIROSHI ET AL: "Heterogeneous Nuclear Ribonucleoprotein A2 Participates in the Replication of Japanese Encephalitis Virus through an Interaction with Viral Proteins and RNA", JOURNAL OF VIROLOGY, vol. 85, no. 21, 1 November 2011 (2011-11-01), US, pages 10976 - 10988, XP093023265, ISSN: 0022-538X, Retrieved from the Internet <URL:https://journals.asm.org/doi/pdf/10.1128/JVI.00846-11> DOI: 10.1128/JVI.00846-11
- PATRICIA FIELDING ET AL: "Heterogeneous Nuclear Ribonucleoprotein A2/B1 Up-Regulation in Bronchial Lavage Specimens: A Clinical Marker of Early Lung Cancer Detection 1", CLINICAL CANCER RESEARCH, vol. 5, 1 December 1999 (1999-12-01), pages 4048 - 4052, XP055222455
- GALAN C ET AL: "Host cell proteins interacting with the 3' end of TGEV coronavirus genome influence virus replication", VIROLOGY, ELSEVIER, AMSTERDAM, NL, vol. 391, no. 2, 1 September 2009 (2009-09-01), pages 304 - 314, XP026458388, ISSN: 0042-6822, [retrieved on 20090705]
- KAMMA H ET AL: "Molecular characterization of the hnRNP A2/B1 proteins: Tissue-specific expression and novel isoforms", EXPERIMENTAL CELL RESEARCH, ELSEVIER, AMSTERDAM, NL, vol. 246, no. 2, 1 February 1999 (1999-02-01), pages 399 - 411, XP002369363, ISSN: 0014-4827, DOI: 10.1006/EXCR.1998.4323
- HUMPHRIES FIACHRA ET AL: "hnRNPA2B1: Fueling Antiviral Immunity from the Nucleus", MOLECULAR CELL, ELSEVIER, AMSTERDAM, NL, vol. 76, no. 1, 3 October 2019 (2019-10-03), pages 8 - 10, XP085849382, ISSN: 1097-2765, [retrieved on 20191003], DOI: 10.1016/J.MOLCEL.2019.09.021
- ZHANG XINGLI ET AL: "hnRNPA2B1: a nuclear DNA sensor in antiviral immunity", CELL RESEARCH, SPRINGER SINGAPORE, SINGAPORE, vol. 29, no. 11, 30 August 2019 (2019-08-30), pages 879 - 880, XP036920716, ISSN: 1001-0602, [retrieved on 20190830], DOI: 10.1038/S41422-019-0226-8
- WANG LEI ET AL: "Nuclear hnRNPA2B1 initiates and amplifies the innate immune response to DNA viruses", SCIENCE, vol. 365, no. 6454, 16 August 2019 (2019-08-16), US, XP093023318, ISSN: 0036-8075, DOI: 10.1126/science.aav0758
- CHANG, C.K. ET AL.: "Cellular hnRNP A2/B1 interacts with the NP of influenza A virus and impacts viral replication", PLOS ONE, vol. 12, no. 11, 16 November 2017 (2017-11-16), XP055774137, DOI: 20200330151521X
- CHANG, C.K. ET AL.: "Cellular hnRNP A2/B1 interacts with the NP of influenza A virus and impacts viral replication", PLOS ONE, vol. 12, no. 11, 16 November 2017 (2017-11-16), XP055774137, DOI: 20200330151601Y
- WANG, Y.M. ET AL.: "hnRNP A2/B1 interacts with influenza A viral protein NS1 and inhibits virus replication potentially through suppressing NS1 RNA/protein levels and NS1 mRNA nuclear export", VIROLOGY, vol. 449, 20 January 2014 (2014-01-20), XP028669474, DOI: 20200330151816X
- WANG, Y.M. ET AL.: "hnRNP A2/B1 interacts with influenza A viral protein NS1 and inhibits virus replication potentially through suppressing NS1 RNA/protein levels and NS1 mRNA nuclear export", VIROLOGY, vol. 449, 20 January 2014 (2014-01-20), XP028669474, DOI: 20200330151828Y
- CUI, H.Q. ET AL.: "Up-regulation and subcellular localization of hnRNP A2/B1 in the development of hepatocellular carcinoma", BMC CANCER, vol. 10, 6 July 2010 (2010-07-06), XP021075178, DOI: 20200330152111Y
- DATABASE Protein 30 June 2018 (2018-06-30), ANONYMOUS: "heterogeneous nuclear ribonucleoproteins A2/B1 isoform 2 [Mus musculus]", XP055890471, retrieved from Genbank Database accession no. NP_872591
- DATABASE Protein 23 December 2018 (2018-12-23), ANONYMOUS: "heterogeneous nuclear ribonucleoproteins A2/B1 isoform A2 [Homo sapiens]", XP055890473, retrieved from Genbank Database accession no. NP_002128

## Description

### Technical field

The present disclosure relates to the field of biotechnology and medicine. Specifically, the present disclosure relates to the effects, mechanism of action, application and use of the nuclear protein hnRNPA2B1 (i.e., heterogeneous nuclear ribonucleoprotein A2B1) in preventing or treating infection related diseases or symptoms and controlling injury caused by infection.

### Background

Infection, especially viral infection, is a common clinical disease with great harm. At first, the molecular mechanism of antiviral infection in body was not fully understood. With the discovery of a type of cytokines, interferons, the molecular biological basis of natural immune cells and secondary acquired immune cells induced by virus infection and their function have been recognized gradually.

Interferon (IFN) is the general name for a family of cytokines with strong antiviral function. In 1957, Professor Alick Isaac and Professor Jean lindenmann discovered a component during a study on infection of chicken embryos with influenza virus. This component was found to be able to significantly prevent the proliferation of influenza virus, and was named interferon (Isaac, A. et al., Proc R Soc Lond B Biol Sci. 1957; 927:258-267). Thereafter, cytokines of the interferon family have gradually been found to have very broad spectrum and effective antiviral effects. At present, interferon has been widely used in clinical to defense against virus infection and treatment of various diseases caused by virus infection.

Different pathogens that can infect the body generally share similarities in structural or molecular characteristics, such as relatively conservative sequence characteristics among the viruses, or constitutively expressed molecules shared by pathogenic microorganisms. These structures and characteristics of similarity are collectively referred to as pathogen associated molecular patterns (PAMPs). The cell surface receptors that are recognized by the PAMPs, or recognize the PAMPs are called pattern recognition receptors (PRRs). For example, PPRs that specifically recognize RNA viruses include RNA helicase retinoic acid-inducible gene I (RIG-I, also known as DDX58) and melanoma differentiation-associated gene 5 (MDA5, also known as IFIH1), and they mainly recognize the AT element-rich part of the RNA viruses that can infect cells.

Usually, once DNA viruses enter host cells, they release and replicate their genomic DNA in the host nucleus. However, the mechanism of recognition of pathogen derived DNAs in the nucleus remains unknown. So far, only one protein, gamma-interferon-inducible protein 16 (IFI16) is found to be able to recognize viral DNA in nucleus and activate the production of IFN-I and inflammatory reaction.

Many proteins have been found to be able to recognize viral DNA and induce production of IFN-α/β, such as RNA polymerase III, IFI16, DAI, LRRFIP1, LSm14A, MRE11, DNA-PK, HMGBs, DDX41, and cyclic GMP AMP (cGAMP) synthase (cGAs) (Goubau, D., et al.; Immunity, 2013; 38, 855-869). After recognizing an virus, these PPRs induce the production of a large amount of type I interferons by stimulating the immune response signalling pathway in the host, so as to quickly establish the body's first line of defense against infection and prevent the infection of the pathogen. However, only cytoplasmic CGAs and DNA-PK have been confirmed as DNA recognition receptors via *in vivo* experiments in mice. There are also several proteins involved in DNA virus induced inflammatory reaction, including AIM2, IFI16, Rad50 and Sox2. Therefore, the study on intranuclear DNA recognition needs to be deepened in order to comprehensively and clearly understand the natural immune response against DNA viruses, especially to find a mechanism that associates the recognition of intranuclear exogenous DNA with the activation of extranuclear natural immune signalling.

Heterogeneous nuclear riboprotein A2B1 (hnRNP-A2B1) belongs to the hnRNP family which includes at least 20 proteins abundantly expressed and others that are less abundantly expressed, and also belongs to RNA binding proteins (RBPs). These molecules are involved in mRNA splicing, transport and other mRNA and miRNA associated processes. hnRNP-A2B1 contains two RNA/DNA-recognition motifs (RRMs) in series at the N-terminal, indicating the DNA binding ability of it. At present, it has been found that hnrNPA2B1 plays an important role in various biological activities. For example, hnRNPA2B1 can directly bind to a pri-miRNA, and promote the processing of the mature form of the pri-miRNA together with DGCR8, one of miRNA microprocessing protein complexes. In addition, hnRNPA2B1 is associated with the progression of many tumors, including lung cancer (Fielding P. et al., Clin Cancer Res. 1999 Dec; 5(12): 4048-52), breast cancer (Cui H. et al., Breast Cancer Res Treat. 2001 Apr; 66(3): 217-24), hepatocellular carcinoma (Zhou J. et al., BMC Cancer. 2010 Jul 6; 10: 356), etc. However, so far, the role of hnRNPA2B1 protein in immune responses and prevention of viral infections is not clear.

Chang et al (2017; PLoS ONE 12(11): e0188214) discloses that cellular hnRNP A2/B1 interacts with the NP of influenza A virus and impacts viral replication. Wang et al (2014; Virology, vol. 449, 20) discloses that hnRNP A2/B1 interacts with influenza A viral protein NS1 and inhibits virus replication potentially through suppressing NS1 RNA/protein levels and NS1 mRNA nuclear export. Cui et al (2010; BMC Cancer, vol. 10) discloses up-regulation and subcellular localization of hnRNP A2/B1 in the development of hepatocellular carcinoma. DATABASE Protein (2018) discloses heterogeneous nuclear ribonucleoproteins A2/B1 isoform 2 [Mus musculus]. DATABASE Protein (2018) discloses heterogeneous nuclear ribonucleoproteins A2/B1 isoform A2 [Homo sapiens]. Katoh Hiroshi et al (2011; Journal of Virology, vol. 85, no. 21, pages 10976-10988) discloses heterogeneous Nuclear Ribonucleoprotein A2 Participates in the replication of Japanese encephalitis virus through an interaction with viral proteins and RNA. US 2003/068803 A1 discloses purification of functional ribonucleoprotein complexes. Galan et al (2009; Virology, Elsevier, Amsterdam, NL, vol. 391, no. 2, pages 304-314) discloses host cell proteins interacting with the 3' end of TGEV coronavirus genome influence virus replication. Kamma et al (1999; Experimental Cell Research, Elsevier, Amsterdam, NL, vol. 246, no. 2, pages 399-411) discloses molecular characterization of the hnRNP A2/B1 proteins: tissue-specific expression and novel isoforms.

To sum up, there is an urgent need in the art to develop an immunologically active agent that can recognize viral DNA in the nucleus, initiate interferon production, enhance antiviral effect, effectively prevent viral infection, and control injury caused by viral infection.

### Summary of invention

The invention is as set out in the appended claims.

One of the main purposes of the present disclosure is to provide the use of an hnRNPA2B1, a nucleic acid molecule encoding said protein, promoters or inhibitors thereof in anti-infection, and further to provide use of them in the treatment or prevention of an infectious disease and associated condition or symptom. The agents, pharmaceutical compositions or kits of the present disclosure can be used to effectively prevent infection and control the occurrence of infectious diseases.

In some aspects of the present disclosure, provided is the use of a heterogeneous nuclear ribonucleoprotein A2B1 (hnRNPA2B1), a nucleic acid molecule encoding said protein, and promoters or inhibitors thereof in the manufacture of a product for the treatment and/or prevention of an infectious disease and/or an infection associated condition and/or symptom.

In some aspects of the present disclosure, provided is a method of preventing and/or treating an infectious disease and/or an infection associated condition and/or symptom, which comprises administrating a patient in need thereof a prophylactically and/or therapeutically effective amount of heterogeneous nuclear ribonucleoprotein A2B1 (hnRNPA2B1), a nucleic acid molecule encoding said protein, a promoter or an inhibitor thereof.

In some aspects of the present disclosure, provided is heterogeneous nuclear ribonucleoprotein A2B1 (hnRNPA2B1), a nucleic acid molecules encoding said protein, a promoter or an inhibitor thereof, for use in the prevention and/or treatment of an infectious disease and/or an infection associated condition and/or symptom.

In some aspects of the present disclosure, provided is the use of heterogeneous nuclear riboprotein A2B1 (hnrnpa2b1), a nucleic acid molecule encoding said protein, and a promoter thereof for improving the level of interferon (such as type I interferon, such as IFN-α and/or IFN-β). In some embodiments, hnRNPA2B1, a nucleic acid molecule encoding said protein, or a promoter thereof is for use in the promotion of an immune response, to prevent and/or treat an infection.

In some aspects of the present disclosure, provided is the use of an inhibitor of heterogeneous nuclear riboprotein A2B1 (hnRNPA2B1) or an inhibitor of a nucleic acid molecule encoding the protein for reducing the level of interferon (such as type I interferon, such as IFN-α and/or IFN-β). In some aspects of the present disclosure, an inhibitor of hnRNPA2B1 or an inhibitor of a nucleic acid molecule encoding hnRNPA2B1 protein are used for the inhibition of excessive immune response. In some aspects of the present disclosure, the ability of an inhibitor of hnRNPA2B1 or hnRNPA2B1 coding sequences in inhibiting cells' resistance to viral infection promotes the replication of viruses in the cells.

In some embodiments, the hnRNPA2B1 is selected from:
(a) a polypeptide having the amino acid sequence set forth in SEQ ID NO: 2 or SEQ ID NO: 4; or
(b) a protein or polypeptide that has a homology or sequence identity (for example, a homology of more than 80% homology or a sequence identity of more than 80%, such as 80%, 85%, 90%, 95%, 98%, 99%) with the amino acid sequence set forth in SEQ ID NO: 2 or SEQ ID NO: 4, and has infection inhibitory activity; or
(c) a protein or polypeptide that is derived from the amino acid sequence of (a) or (b) with substitution, deletion or addition of one or more amino acids in the amino acidseqence of (a) or (b), and prevents and/or treats an infectious disease and/or an infection associated condition and/or syndrome.

In some embodiments, hnRNPA2B1 is a naturally occurring and purified protein, a chemically synthesized product, or produced from a prokaryotic or eukaryotic host through recombination techniques. The host is selected from bacteria, yeast, higher animal and mammalian cells. Preferably, the hnRNPA2B1 is a human hnRNPA2B1.

In some embodiments, hnRNPA2B1 is a polypeptide of the amino acid sequence set forth in SEQ ID NO: 2 or SEQ ID NO: 4.

In some embodiments, the nucleic acid molecule encoding hnRNPA2B1 is selected from:
(i) a nucleic acid molecule having the nucleotide sequence set forth in SEQ ID NO: 1 or SEQ ID NO: 3; or
(ii) a molecule that hybridizes with the nucleotide sequence defined in (i) under a strict condition;
(iii) a nucleic acid molecule, which has a homology or sequence identity (e.g. a homology of more than 80% or a sequence identity of more than 80%, such as 80%, 85%, 90%, 95%, 98%, 99%) with the nucleotide sequences set forth in SEQ ID NO:1 or SEQ ID NO:3, and encodes a protein or polypeptide of activity in preventing and/or treating an infectious disease and/or an infection associated condition and/or symptom;
(iv) a nucleic acid molecule which has substitution, deletion or addition of one or more nucleotides in the nucleotide sequence of (i) or (ii) or (iii), and encodes a protein or polypeptide of activity in preventing and/or treating an infectious disease and/or an infection associated condition and/or symptom.

In some embodiments, the nucleotide sequence of the nucleic acid molecule encoding hnRNPA2B1 is set forth in SEQ ID NO: 1 or SEQ ID NO: 3.

In some embodiments, the promoter is selected from: agents that increase the protein level of hnRNPA2B1 or promote the function of hnRNPA2B1, such as an overexpression vector of hnRNPA2B1 or hnRNPA2B1 coding sequence; exogenous hnRNPA2B1; a naked DNA of hnRNPA2B1 coding sequence; a liposome encapsulated DNA of hnRNPA2B1 coding sequence; hnRNPA2B1 precursor protein or conjugate or complex that can be transformed into hnRNPA2B1 *in vivo.*

In some embodiments, the inhibitor is selected from: an antibody against hnRNPA2B1 or against a nucleic acid molecule encoding the protein, siRNA (e.g., an siRNA with a sequence as set forth in any one of SEQ ID NOs: 5-8), an miRNA, an antisense oligonucleotide, an antagonist, a blocker.

In some embodiments, the infection is a DNA-involved and/or DNA-mediated infection.

In some embodiments, the infection is a virus infection or an DNA involved infection such as those caused by bacteria and fungi, or a combination thereof, such as DNA virus infection, such as infection caused by one or more viruses selected from the group consisting of: herpes simplex virus, hepatitis B virus, adenovirus, poxvirus, small DNA virus and adeno-associated virus.

In some embodiments, the infection associated diseases and/or symptoms are one or more selected from the group consisting of: pathological injury caused by infection; insufficient or excessive production of cytokines such as interferon after infection; endotoxic shock or death; inflammatory injury of organs; multiple organ failure, for example, the organ is selected from: liver, spleen, brain, kidney, heart, lung, stomach and intestine; chronic inflammatory diseases caused by viral infections (such as autoimmune diseases such as inflammatory bowel disease, rheumatoid arthritis, systemic lupus erythematosus, chronic nephritis, tuberculosis, chronic gastrointestinal diseases). Preferably, chronic inflammatory diseases and/or symptoms caused by viral infections include autoimmune diseases such as inflammatory bowel disease, rheumatoid arthritis, systemic lupus erythematosus, chronic nephritis, tuberculosis, and chronic gastrointestinal diseases.

In some embodiments, the product is a pharmaceutical composition or kit, for example, in a form that is suitable for the administration selected from the group consisting of oral administration, injection (such as direct naked DNA or protein injection, liposome encapsulated DNA or protein injection), gold coated gene gun bombardment, reproduction defective bacteria carrying plasmid DNA, replication deficient adenovirus carrying a protein encoded by a target DNA method or target gene, electroporation, nasal administration, pulmonary administration, oral administration, transdermal administration and intratumoral administration.

In some embodiments, the product also contains other agents for prevention and/or treatment of an infectious disease and/or an infection associated condition and/or symptom, such as one or more of clinically common antibiotics (including β-lactams (penicillins and cephalosporins), aminoglycosides, tetracyclines, chloramphenicols, macrolides, antifungal antibiotics and anti-tuberculosis antibiotics).

In some aspects of the present disclosure, a product (e.g., a pharmaceutical composition or kit) is provided, which comprises:
(A) a therapeutically or prophylactically effective amount of hnRNPA2B1 described herein, a nucleic acid molecule encoding the protein, a promoter thereof and/or an inhibitor thereof;
(B) a pharmaceutically or immunologically acceptable carrier or excipient;
(C) optionally, one or more other actives for preventing or treating an infectious disease and its associated diseases and/or symptoms.

In some embodiments, the product comprises 0.001-99.9wt% of hnRNPA2B1 or an hnRNPA2B1-encoding nucleic acid molecule, a promoter or an inhibitor thereof, based on the total weight of the product.

In some embodiments, the product comprises 1-95 wt%, preferably 5-90 wt%, more preferably 10-80 wt% of the hnRNPA2B1 or an hnRNPA2B1-encoding nucleic acid molecule, a promoter or an inhibitor thereof, based on the total weight of the product. The remainder can be an agent such as a pharmaceutically acceptable carrier and other additives.

In some embodiments, further actives that can regulate the inhibition of a viral infection are administered before, concurrent with or after the administration of the product of the present disclosure. The further actives have the activity of preventing or treating diseases associated with viral infection, injury caused by infection, chronic inflammatory diseases caused by infection and/or symptoms thereof. The viral infection is caused by one or more selected from the group consisting of: herpes simplex virus, hepatitis B virus, adenovirus and poxvirus.

In some embodiments, the other actives include, but are not limited to, one or more selected from the group consisting of: clinically common antibiotics, including β-lactams (penicillins and cephalosporins), aminoglycosides, tetracyclines, chloramphenicols, macrolides, antifungal antibiotics and anti-tuberculosis antibiotics; clinically common antiviral drugs (tricyclic amines, pyrophosphates, protease inhibitors, nucleoside drugs, interferon, antisense oligonucleotides, etc.); clinically common immunosuppressants (including glucocorticoid, cyclophosphamide, chloroquine, cyclosporine A, Tripterygium wilfordii, traditional Chinese medicine preparation and an anti-TNF monoclonal antibody).

In some aspects of the present disclosure, provided is a method for screening an agent that has an anti-infection activity by promoting hnRNPA2B1, which comprises:
(A) treating infected cells, tissues or animals with candidate agents;
(B) detecting the level of hnRNPA2B1 or the nucleic acid molecule encoding the protein in the cells, tissues or animals; and
(C) if the level of hnRNPA2B1 or the nucleic acid molecule encoding the protein is higher than that before the treatment with the candidate agent or higher than that in the normal control, it is indicated that the candidate agent has an anti-infection activity by promoting hnRNPA2B1.

### Description of drawings

The present disclosure will be further described below in combination with the accompanying drawings, wherein these drawings are only for the purpose of illustrating embodiments of the present disclosure, not to limit the scope of the present disclosure.
**FIG. 1****:** After the mouse primary peritoneal macrophages were infected with HSV-1, hnRNPA2B1 directly recognized and was bound to HSV-1 DNA. This figure shows the detection of HSV-1 DNA by nucleic acid electrophoresis after PCR.
**FIG. 2****:** The mouse macrophages were transfected with interfering RNA (siRNA) for hnRNPA2B1, resulting in a decreased transcription level of type I interferon after HSV-1 infection. This figure shows IFN-α (Ifna4), IFN-β (IFnb1) mRNA levels detected by fluorescence quantitative PCR (***, P<0.001; **, P<0.01). UI: uninfected, un-stimulated group.
**FIG. 3****:** The mouse macrophages were transfected with interfering RNA (siRNA) for hnRNPA2B1, resulting in a decreased protein level of IFN-β after HSV-1 infection. This figure shows ELISA analysis (***, P<0.001). UI: uninfected, un-stimulated group.
**FIG. 4****:** The human THP1 cells were transfected with interfering RNA (siRNA) for hnRNPA2B1, resulting in a decreased transcription level of type I interferon after HSV-1 infection. This figure shows the PCR-α (Ifna4), IFN-β (IFnb1) mRNA level detected by fluorescence quantitative PCR (***, P<0.001). UI: uninfected, un-stimulated group.
**FIG. 5****:** Overexpression of hnRNPA2B1 in mouse macrophages resulted in increased protein secretion level of IFN-β after HSV-1 infection. The figure shows ELISA analysis (***, P<0.001).
**FIG. 6****:** Wild-type mice and myeloid cell specific hnRNPA2B1 knockout (Hnrnpa2b1 cKO) mice were subjected to intraperitoneal infection of 7×10⁸ plaque forming units (PFUs) of HSV-1. The IFN-β level in blood was detected by ELISA after 6 hours, and the serum IFN-β level in hnRNP-A2B1 knockout mice was significantly lower than that of wild-type mice (***, P<0.001).
**FIG. 7****:** Wild-type mice and myeloid cell specific hnRNPA2B1 knockout (Hnrnpa2b1 cKO) mice were subjected to intraperitoneal infection of 7×10⁸ plaque forming unit (PFU) of HSV-1. The virus titer in the brain was detected by plaque test after 4 days, and the virus titer in the brain of hnRNP-A2B1 knockout mice was significantly higher than that of wild-type mice (***, P<0.001).
**FIG. 8****:** Wild-type mice and myeloid cell specific hnRNPA2B1 knockout (Hnrnpa2b1 cKO) mice were subjected to intraperitoneal infection of 7×10⁸ plaque forming unit (PFU) of HSV-1. The survival of the hnRNPA2B1 knockout mice was decreased significantly (P<0.001).
**FIG. 9****:** Knockout of the expression of hnRNPA2B1 resulted in the decreased transcription level of type I interferon. This figure shows the IFN-β MRNA level detected by fluorescence quantitative PCR (**, P<0.01). UI: uninfected, un-stimulated group.

### Embodiments

In this disclosure, through extensive research and animal model tests, it is found that hnRNPA2B1 can effectively inhibit viral infection, improve organ function and improve survival in infectious diseases. On this basis, the present disclosure has been completed.

The present disclosure has studied the efficacy and function of the novel immunomodulatory molecule hnRNPA2B1 in anti-infection, and demonstrated the therapeutic and protective effects of the molecule on infected animals. Experiments showed that: 1) hnRNPA2B1 can bind to virus DNA after virus infection; 2) interfering with the expression of hnRNPA2B1 inhibits the production of type I interferon after virus infection; 3) hnRNPA2B1 knockout can increase HSV-1 infection in mouse brain and increase mortality; 4) overexpression of hnRNPA2B1 can promote the production of type I interferon after virus infection. These experimental results suggest that hnRNPA2B1 has the application prospect of treating DNA involved and/or mediated infections (such as viral infectivity, such as HSV-1, ADV infection, etc.). Accordingly, the present disclosure provides methods and strategies for applying anti-infective molecule hnRNPA2B1 to inhibit infection or for the prevention and treatment of infectious diseases, especially for controlling viral infection, such as liver injury caused by viral infection.

All the numerical ranges provided herein are intended to clearly include all values falling between the endpoints of the range and the numerical range between them. The features mentioned in the present disclosure or the features mentioned in the examples may be combined.

As used herein, "contain", "have" or "comprise" includes "include", "consist mainly of", "consist essentially of", and "consist of"; "mainly composed of", "basically composed of" and "composed of" belong to the subordinate concepts of "contain", "have" or "comprise".

### hnRNPA2B1 protein (polypeptide)

As used herein, the terms "hnRNPA2B1 protein (polypeptide)" and "hnRNPA2B1" can be used interchangeably to refer to heterogeneous nuclear riboprotein A2B1. The hnRNPA2B1 protein of the present disclosure can be a protein encoded by the sequence of SEQ ID NO: 1 (full-length sequence of human cDNA) or SEQ ID NO: 3 (mouse CDS sequence), or a homologous sequence (for example, the homologous sequence of hnRNPA2B1 can be obtained through a database or comparison software known in the art), a variant or a modified form of these proteins that can promote the expression of interferon. For example, the hnRNPA2B1 protein may be selected from: (a) the amino acid sequence set forth in SEQ ID NO: 2 or SEQ ID NO: 4; or (b) a protein or polypeptide derived from (a) that has substitution, deletion or addition of one or more amino acids in the amino acid sequence defined in (a), and has the activity of promoting the expression of interferon.

The proteins or polypeptides of the present disclosure may be naturally occurring, purified products, or chemically synthesized products, or produced from prokaryotic or eukaryotic hosts (e.g., bacteria, yeast, higher animal, insect and mammalian cells) using recombination techniques. The hnRNPA2B1 protein or polypeptide of the present disclosure is preferably encoded by human hnRNPA2B1 gene or its homologous gene or family gene.

The variant forms of the protein or polypeptide of the present disclosure include (but are not limited to): deletion, insertion and/or substitution of one or more (usually 1-50, preferably 1-30, more preferably 1-20, most preferably 1-10, such as 1, 2, 3, 4, 5, 6, 7, 8, 9 or 10) amino acids, and addition of one or more (usually less than 20, preferably less than 10, more preferably less than 5) amino acids at the C-terminal and/or N-terminal. For example, in the art, when replacing with amino acids of close or similar properties, the function of a protein or polypeptide is usually not changed. For another example, addition of one or more amino acids at the C-terminal and/or N-terminal usually does not change the function of the protein or polypeptide. For example, the hnRNPA2B1 protein or polypeptide of the present disclosure may or may not include the initial methionine residue but still have the activity of inhibiting infection.

Random mutagenesis can be generated by radiation or exposure to mutagens, or the protein or polypeptide in (b) above can be obtained by site-specific mutagenesis or other known molecular biological techniques. Transgenic animals can be constructed by using the coding sequence encoding the protein or polypeptide, and observed for their resistance to virus infection or improvement in resistance to virus infection to identify the obtained protein or polypeptide.

According to the host used in the recombinant production scheme, the protein or polypeptide of the present disclosure may be glycosylated or non-glycosylated. The term also refers to active fragments and active derivatives of hnRNPA2B1 protein.

The variant forms of the polypeptide include: homologous sequences, conservative variants, allelic variants, natural mutants, induced mutants, proteins encoded by a sequence that can hybridize with hnRNPA2B1 protein coding sequence under highly or lowly strict conditions, and polypeptides or proteins obtained from antiserum against hnRNPA2B1 protein. Other polypeptides, such as fusion proteins containing hnRNPA2B1 protein or fragments thereof, can also be used in the present disclosure. In addition to the almost full-length polypeptide, the present disclosure also includes a soluble fragment of hnRNPA2B1 protein. Usually, the fragment has at least about 10 continuous amino acids, usually at least about 30 continuous amino acids, preferably at least about 50 continuous amino acids, more preferably at least about 80 continuous amino acids, and most preferably at least about 100 continuous amino acids of the hnRNPA2B1 protein sequence.

### A nucleic acid molecule encoding hnRNPA2B1

As used herein, the terms "hnRNPA2B1 gene", "hnRNPA2B1 coding gene", "hnRNPA2B1 protein coding gene" or "nucleic acid molecule encoding hnRNPA2B1" can be used interchangeably, and all refer to a nucleotide sequence encoding hnRNPA2B1 protein or polypeptide described herein, which can be, for example, the nucleotide sequence shown in SEQ ID NO: 1 (human CDS) or SEQ ID NO: 3 (mouse CDS) sequence, molecules hybridized with these sequences under a strict condition, or family gene molecules that are highly homologous to the above molecules, wherein the expression of the gene can promote the production and effect of interferon. The hnRNPA2B1 gene is highly conserved in human and mice. The gene ID for human is 3181 and the gene ID for mouse is 53379.

The hnRNPA2B1 gene of the present disclosure can be selected from: (i) SEQ ID NO: 1, SEQ ID NO: 3; or (ii) a molecule that hybridizes with the sequence defined in (i) under a strict condition and promotes interferon production.

As used herein, the term "strict condition" refers to: (1) hybridization and elution at lower ionic strength and higher temperature, such as 0.2×SSC, 0.1 %SDS, 60°C; or (2) addition of denaturant during hybridization, such as 50% (v/v) formamide, 0.1% fetal bovine serum/0.1% Ficoll, 42 °C, etc; or (3) hybridization that occurs only when the identity between the two sequences is at least 50%, preferably more than 55%, more than 60%, more than 65%, more than 70%, more than 75%, more than 80%, more than 85% or more than 90%, more preferably more than 95%. For example, the sequence may be a complementary sequence of the sequence defined in (a).

The full-length nucleotide sequence or fragment of the hnRNPA2B1 gene of the present disclosure can generally be obtained by PCR amplification, recombination or synthetic methods. For PCR amplification, primers can be designed according to the relevant nucleotide sequences disclosed in the disclosure, especially the open reading frame sequence, and the relevant sequences can be amplified using the commercially available cDNA library or the cDNA library prepared according to the conventional method known to those skilled in the art as a template. When the sequence is relatively long, two or more PCR amplification is usually required, and then the amplified fragments are spliced together in the correct order.

It should be understood that the hnRNPA2B1 gene of the present disclosure is preferably obtained from human. Other genes obtained from other animals that are highly homologous with the human hnRNPA2B1 gene (for example, having a sequence identity of more than 50%, preferably more than 55%, more than 60%, more than 65%, more than 70%, more than 75%, more than 80%, more preferably more than 85%, such as 85%, 90%, 95%, 98% or even 99% or more) are also within the scope of the equivalent embodiments preferably considered in the present disclosure. Methods and tools for aligning sequence identity are also well known in the art, such as BLAST.

### Promoter and inhibitor of hnRNPA2B1 or hnRNPA2B1 coding sequence

The present disclosure also relates to a "promoter" of hnRNPA2B1 or hnRNPA2B1 coding sequence. The term "promoter" or "promoter of hnRNPA2B1 or its coding sequence" can be used interchangeably to refer to agents that can improve the level or activity of hnRNPA2B1 or its coding nucleic acid molecules. Promoters that can be used in the present disclosure include but are not limited to: hnRNPA2B1 expression vector, exogenous hnRNPA2B1, naked DNA of hnRNPA2B1 or its coding sequence, liposome encapsulated DNA of hnRNPA2B1 or its coding sequence, and hnRNPA2B1 protein.

hnRNPA2B1 or hnRNPA2B1 coding sequence or its promoter of the present disclosure can inhibit viral infection, so it can be further used to prevent or treat diseases associated with viral infection, and/or related symptoms caused by viral infection, as well as chronic inflammatory diseases caused by infection, and/or symptoms thereof.

The present disclosure also relates to "inhibitor" of hnRNPA2B1 or hnRNPA2B1 coding sequences. The term "inhibitor" or "inhibitor of hnRNPA2B1 or its coding nucleic acid molecule" can be used interchangeably to refer to an agent that can reduce the level or activity of hnRNPA2B1 or its coding nucleic acid molecule. Inhibitors that can be used in the present disclosure include, but are not limited to, antibodies against hnRNPA2B1 or nucleic acid molecules encoding the protein, siRNA (e.g., siRNA with sequence as set forth in any one of SEQ ID NOs: 5-8), miRNA, antisense oligonucleotides, antagonists and blockers.

The inhibitor of the present disclosure can inhibit excessive anti-infective response (such as interferon production), so that it can be further used to prevent or treat infection associated diseases and/or associated symptoms, for example, chronic inflammatory diseases caused by infection (such as autoimmune diseases such as inflammatory bowel disease, rheumatoid arthritis, systemic lupus erythematosus, chronic nephritis, tuberculosis, chronic gastrointestinal diseases).

### Vector, host and transgenic animal

The present disclosure also relates to a vector containing the hnRNPA2B1 gene, a host cell generated by genetic engineering with the vector, and a transgenic animal with high expression of hnRNPA2B1 obtained through transgene.

Through conventional recombinant DNA technology (Science, 1984; 224:1431), the coding sequence of the present disclosure can be used to express or produce recombinant hnRNPA2B1 protein. Generally, there are the following steps:
(1) Transforming or transducing a suitable host cell with a polynucleotide (or variant) encoding hnRNPA2B1 protein of the present disclosure or with a recombinant expression vector containing the polynucleotide;
(2) Culturing the host cells in a suitable medium;
(3) Isolating and purifying proteins or peptides from the medium or cells.

In the present disclosure, the terms "vector" and "recombinant expression vector" can be used interchangeably to refer to bacterial plasmids, phages, yeast plasmids, animal cell viruses, mammalian cell viruses or other vectors well known in the art. To sum up, any plasmid and vector can be used as long as it can replicate and stabilize in the host. An important feature of expression vectors is that they usually contain origin of replication, promoters, marker genes and translation control elements.

The method well known to those skilled in the art can be used to construct an expression vector containing hnRNPA2B1 coding sequence and appropriate transcription/translation control signal. These methods include *in vitro* recombinant DNA technology, DNA synthesis technology, *in vivo* recombinant technology, etc. The DNA sequence can be effectively connected to an appropriate promoter in the expression vector to guide mRNA synthesis. The expression vector also includes ribosomal binding sites for translation initiation and transcription terminators. The expression system of pcDNA3.1 vector is preferably used in the present disclosure.

In addition, the expression vector preferably comprises one or more selective marker genes to provide phenotypic traits for selecting transformed host cells, such as dihydrofolate reductase for eukaryotic cell culture, neomycin resistance and green fluorescent protein (GFP), or tetracycline or ampicillin resistance for *Escherichia coli.*

Vectors containing above appropriate DNA sequences and appropriate promoters or control sequences can be used to transform appropriate host cells so that they can express proteins or peptides. Host cells can be prokaryotic cells, such as bacterial cells; or lower eukaryotic cells, such as yeast cells; or higher eukaryotic cells, such as animal cells. Representative examples are: *Escherichia coli,* Streptomyces, Agrobacteriuml fungal cells such as yeasts; animal cells, etc. In the present disclosure, E. coli cells and human liver cells are preferably used as host cells.

When the polynucleotide of the present disclosure is expressed in higher eukaryotic cells, the transcription will be enhanced if an enhancer sequence is inserted into the vector. Enhancers are cis acting factors of DNA, usually of about 10 to 300 base pairs, which act on promoters to enhance gene transcription. Those skilled in the art know how to select appropriate vectors, promoters, enhancers and host cells.

The term "transgenic animal" or "transformed animal" in the present disclosure can be used interchangeably to refer to cells, organs, tissues or individuals obtained by conventional transgenic methods that are transferred with the hnRNPA2B1 gene of the present disclosure and stably and highly express hnRNPA2B1 protein or polypeptide.

The recombinant polypeptide in the above method can be expressed in the cell or on the cell membrane or secreted outside the cell. If necessary, the recombinant protein can be separated and purified by various separation methods based on its physical, chemical and other properties. These methods are well known to those skilled in the art. Examples of these methods include, but are not limited to: conventional renaturation, protein precipitation treatment (salting out method), centrifugation, osmosis, over treatment, ultracentrifugation, molecular sieve chromatography (gel filtration), adsorption chromatography, ion exchange chromatography, high performance liquid chromatography and other liquid chromatography techniques and the combination of these methods.

### Product

The present disclosure also provides a product, which may be, for example, a drug, a pharmaceutical composition or a kit containing an effective amount of hnRNPA2B1 of the present disclosure or a nucleic acid molecule encoding hnRNPA2B1, a promoter thereof or an inhibitor thereof, and a pharmaceutically or immunologically acceptable carrier. As used herein, the term "active", "active agent" or "active agent of the present disclosure" can be used interchangeably to refer to hnRNPA2B1 or hnRNPA2B1 coding sequence, its promoter or its inhibitor.

In a preferred embodiment, the product can be used to prevent or treat diseases associated with viral infection, chronic inflammatory diseases caused by viral infection, and/or symptoms thereof; for example, the pharmaceutical compositions disclosed herein can be used to prevent or treat viral infectious diseases which are known to be prevented or treated, such as tissue injury caused by viral infections, inflammatory injury of organs; and multiple organ failure.

As used herein, the term "contain" or "comprise" includes "include", "consist essentially of', and "consist of'. As used herein, the term "pharmaceutically acceptable" ingredient is applicable to humans and/or animals without excessive adverse reactions (such as toxicity, irritation and allergy), i.e. an agent with reasonable benefit/risk ratio. As used herein, the term "effective amount" refers to an amount that can produce function or activity to humans and/or animals and can be accepted by humans and/or animals.

As used herein, the term "pharmaceutically acceptable carrier" refers to a carrier used for the administration of therapeutic agents, including various excipients and diluents. The term refers to drug carriers that are not essential active ingredients themselves and are not excessively toxic after administration. Suitable carriers are well known to those skilled in the art, and a full discussion on pharmaceutically acceptable excipients can be found in Remington's Pharmaceutical Sciences, Mack Pub. Co., N.J. 1991.

A pharmaceutically acceptable carrier in a composition may contain liquids such as water, brine, glycerol and ethanol. In addition, there may be auxiliary substances in these carriers, such as fillers, disintegrating agents, lubricants, flow aids, effervescent agents, wetting agents or emulsifiers, flavoring agents, pH buffer substances, etc. Generally, these substances can be prepared in a non-toxic, inert and pharmaceutically acceptable aqueous carrier medium, wherein the pH is usually about 5-8, preferably about 6-8.

The active agent in the product of the present disclosure accounts for 0.001-99.9 wt% of the total weight of the composition, preferably 1-95 wt%, more preferably 5-90 wt%, more preferably 10-80 wt% of the total weight of the composition. The remainder is pharmaceutically acceptable carriers and other additives.

As used herein, the term "unit dosage form" refers to preparing the product of the present disclosure into a dosage form required for single administration for convenience of administration, including but not limited to various solid agents (such as tablets), liquid agents, capsules and sustained-release agents.

In another preferred embodiment of the present disclosure, the product is in a unit dosage form or multiple dosage form, and the content of the active agent is 0.01-2000 mg/dose, preferably 0.1-1500 mg/dose, more preferably 1-1000 mg/dose. In another preferred example of the present disclosure, 1 to 6 doses of the composition of the present disclosure are administrated every day, preferably 1 to 3 doses; most preferably, the daily dose is 1 dose.

It should be understood that the effective dose of active agents such as hnRNPA2B1 protein or its coding sequence used may vary with the severity of the subject to be administered or treated. The specific condition is determined according to the individual condition of the subject (such as the subject's weight, age, physical condition and the effect to be achieved), which can be determined by a skilled physician.

The products of the present disclosure may be solid (such as granules, tablets, lyophilized powder, suppositories, capsules, sublingual tablets) or liquid (such as oral liquid) or other suitable shapes. The route of administration can be: (1) direct naked DNA or protein injection; (2) connection of the cDNA, mRNA and protein of hnRNPA2B1 to transferrin/poly L-lysine complex to enhance its biological effects; (3) complexes fromed with cDNA, mRNA and protein and positively charged lipids to overcome the difficulty of crossing the cell membrane caused by the negative charge of phosphate skeleton; (4) entrance into cells after encapsulation of cDNA, mRNA and protein with liposomes, which can not only facilitate the entry of macromolecules, but also avoid the hydrolysis of extracellular enzymes; (5) the combination of cDNA, mRNA and protein with cholesterol, which increases the cytoplasmic retention time by 10 times; (6) immunoliposomes to specifically transport cDNA, mRNA and protein to target tissues and cells; (7) transfection of cDNA, mRNA and protein into transfer cells (such as fibroblasts) *in vitro,* which can also better load hnRNPA2B1 associated drugs into target cells; (8) electroporation, which inrtoduces cDNA, mRNA and protein into target cells with current.

In addition, the products of the disclosure can also contain other active agents for improving and treating viral infectious diseases. The other active agents are selected from the group consisting of: one or more of clinically common antibiotics (including β-lactams (penicillins and cephalosporins), aminoglycosides, tetracyclines, chloramphenicols, macrolides, antifungal antibiotics and anti-tuberculosis antibiotics).

The nucleotide and protein drugs associated with hnRNPA2B1 of the present disclosure can be applied in combination with each other, and can also be applied in combination with other drugs and therapeutic means for the prevention and treatment of bacterial infectious diseases.

The references to the methods of treatment by therapy or surgery or in vivo diagnosis methods in this description are to be interpreted as references to compounds, pharmaceutical compositions and medicaments for use in those methods

### Examples

The present disclosure is further described below in combination with specific examples. It should be understood that these examples are used only to illustrate the present disclosure and not to limit the scope of the present disclosure.

For the experimental methods without specific conditions noted in the following examples, conventional methods in the art can be adopted, for example, refer to Molecular Cloning: A Laboratory Manual, Third Edition, New York, Cold Spring Harbor Laboratory Press, 1989 or according to the conditions recommended by the supplier. DNA sequencing method is a conventional method in the art and can also be tested by commercial companies.

Unless otherwise stated, percentages and portions are calculated by weight. Unless otherwise defined, all professional and scientific terms used herein have the same meaning as those familiar to those skilled in the art. In addition, any method and material similar or equal to the recorded content can be applied to the method of the present disclosure. The preferred methods and materials described herein are for demonstration purposes only.

**Example 1: hnRNPA2B1 directly recognizes DNA derived from nuclear pathogens.**

Obtaining the primary peritoneal macrophages of mice: C57BL/6 mice (6-8 weeks old, female, Shanghai Bikai Experimental Animal Co., Ltd.) were intraperitoneally injected with 2 ml of 3% mercaptoacetate (Sigma Aldrich) solution once. Three days later, the mice were sacrificed by cervical dislocation, and the abdominal cavity was washed with serum-free medium, sucked out and centrifuged to obtain cells.

The primary peritoneal macrophage cells were cultured in DMEM medium. After HSV-1 (strain F, ATCC) infection for 2 hours, nuclear protein was isolated and immunoprecipitated by anti-hnRNPA2B1 antibody (Santa Cruz) or control antibody (IgG, Santa Cruz). HSV-1 DNA was detected by PCR.

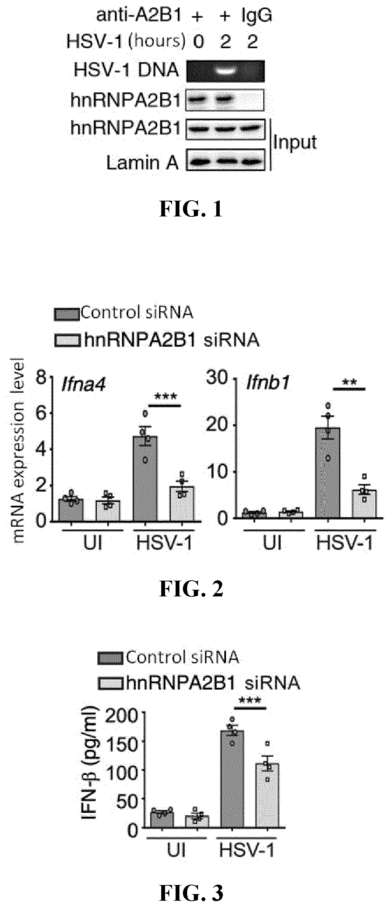

The binding of hnRNPA2B1 to HSV-1 DNA is shown in FIG. 1.

The results showed that HSV-1 DNA was bound to hnRNPA2B1 protein precipitated by anti-hnRNPA2B1 antibody 2 hours after infection.

The results showed that hnRNPA2B1 was bound to HSV-1 DNA during infection.

### Example 2: Interfering with the expression of hnRNPA2B1 inhibits type I interferon induced by HSV-1 infection.

Primary peritoneal macrophage cells were cultured in DMEM medium and human THP1 cells (ATCC) were cultured in 1640 medium. The cells were transfected with small interfering RNA for hnRNPA2B1 (hnRNPA2B1 siRNA) and mock control (control siRNA) (transfection reagent INTERFERin was purchased from Polyplus company).

Small interfering RNA for hnRNPA2B1 (hnRNPA2B1 siRNA) and mock control (control siRNA) were purchased from Genephama. The sequences of mouse hnRNPA2B1 siRNA are set forth in SEQ ID NO: 5 and SEQ ID NO: 6, the sequences of human hnRNPA2B1 siRNA are set forth in SEQ ID NO: 7 and SEQ ID NO: 8, and, the sequences of control siRNA are set forth in SEQ ID NO: 9 and SEQ ID NO: 10. During synthesis, 2 dTs were added at 3' to make the sequence more stable. The specific sequences are as follows:
hnRNPA2B1 siRNA sequences:
Mouse hnRNPAB1 siRNA:
   5'- GAGGAAAUUAUGGAAGUGGTT-3' (sense, SEQ ID NO: 5);
   5'-CCACUUCCAUAAUUUCCUCCT-3' (anti-sense, SEQ ID NO: 6).
Human hnRNPAB1 siRNA:
   5'- CUUUGGUGGUAGCAGGAACTT-3' (sense, SEQ ID NO: 7);
   5'-GUUCCUGCUACCACCAAAGTT-3' (anti-sense, SEQ ID NO: 8).
Control siRNA sequences:
   5'-UUCUCCGAACGUGUCACGUTT-3' (sense, SEQ ID NO: 9);
   5'-ACGUGACACGUUCGGAGAATT-3' (anti-sense, SEQ ID NO: 10).

Mouse peritoneal macrophages (2×10⁵ cells/well) and human THP1 cells (1×10⁶ cells/well) 48 hours after transfection were stimulated by HSV-1 (MOl, 10), the adherent cells were collected 7 hours after infection, the total RNA of the cells was extracted, and the transcriptional level of type I interferon (IFN-α, IFN-β) was detected by real-time fluorescence quantitative PCR; the cell culture supernatant was collected 18 hours after infection, and the protein level of IFN-β was detected by ELISA.

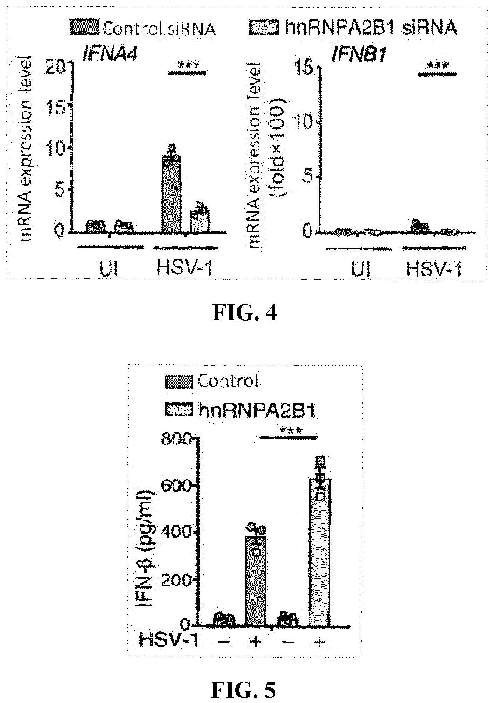

The transcription level of type I interferon (IFN-α, IFN-β) is shown in FIGs. 2 and 4, and the protein level is shown in FIG. 3. In FIGs. 2 and 3, mouse hnRNPAB1 siRNA was used for mouse cells; in FIG. 4, human hnRNPAB1 siRNA was used for human cells.

The results showed that transfection of small interfering RNA for hnRNPA2B1 into mouse macrophages can significantly inhibit the production of type I interferon induced by HSV-1 infection.

The results showed that interfering with the expression of hnRNPA2B1 resulted in the decrease of type I interferon induced by HSV-1 infection, suggesting that hnRNPA2B1 may play an important role in anti-infection.

### Example 3: Overexpression of hnRNPA2B1 inhibits secretion of IFN-β in cells.

Firstly, the cDNA of hnRNPA2B1 (NM_182650) was introduced into eukaryotic expression vector pcDNA3.1 plasmid (Addgene) to construct hnRNPA2B1 expression vector.

Mouse peritoneal macrophages were transfected with hnRNPA2B1 expression vector at the density of 1µg/ml, and fresh DMEM medium was replaced 48 hours later.

Cells with a density of 4×10⁵ cells/ml were infected with HSV-1 (MOl, 10). After 12 hours, the cell culture supernatant was collected, and the IFN-β level in the cell culture supernatant was detected with an ELISA kit.

The secretion of IFN-β is shown in FIG. 5.

The results showed that transfecting cells with hnRNPA2B1 plasmid canpromote HSV-1-induced IFN-β production.

The results showed that overexpression of hnRNPA2B1 can promote the production of IFN-β induced by HSV-1 infection, indicating that hnRNPA2B1 might play an important role in anti-infection.

### Example 4: Knockout of hnRNPA2B1 inhibits in vivo IFN-βlevel in serum induced by HSV-1 infection.

hnRNPA2B1 myeloid cell conditional knockout mice (Shanghai Model Organisms Center, Inc., the construction method may refer to Jnyner AL. Gene Targeting, Oxford University Press.1994) were constructed, wherein IoxP sequence that can be specifically recognized by CRE endonuclease is added to exon 2-6 of *Hnrnpa2b1* gene. *Hnrnpa2b1*^{fl/fl} mice were backcrossed with C57BL/6 background and hybridized with *Lyz2-Cre* mice, so as to realize the specific knockout of exons 2-6 of *Hnrnpa2b1* gene in myeloid cells. *Hnrnpa2b1*^{fl/fl}*Lyz2-Cre*^{+/-} mice and *Hnrnpa2b1*^{fl/fl}*Lyz2-Cre*^{*-*/*-*} mice were crossed to produce myeloid cell specific hnRNPA2B1 knockout (hnrnpa2b1 cKO) mice and littermate control wild-type mice, which were raised in a special pathogen free clean level (SPF) environment.

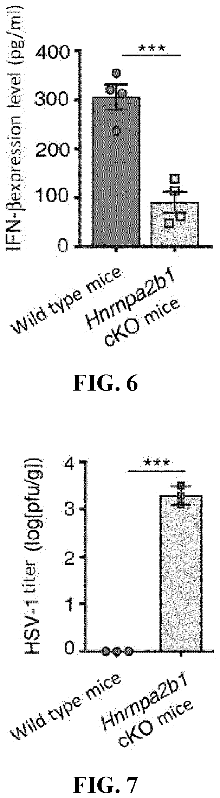

Wild type mice and myeloid cell specific hnRNPA2B1 knockout (hnrnpa2b1 cKO) mice were intraperitoneally infected with 7×10⁸ plaque forming units (PFU) of HSV-1, blood was taken 6 hours later, serum was separated, and IFN-β level in blood was detected by ELISA. The test results are shown in FIG. 6.

The results showed that the serum IFN-β level of hnRNP-A2B1 knockout mice was significantly lower than that of wild-type mice (***, *P*<0.001).

The results showed that hnRNPA2B1 knockout inhibited IFN-β production induced by HSV-1 virus infection, indicating that hnRNPA2B1 may play an important role in anti-infection.

### Example 5: Knockout of hnRNPA2B1 promotes the replication of HSV-1 in mouse brain.

hnRNPA2B1 myeloid cell conditional knockout mice were constructed (as Example 4). Wild type mice and myeloid cell specific hnRNPA2B1 gene knockout (*Hnrnpa2b1* cKO) mice were intraperitoneally infected with 7×10⁸ plaque forming units (PFU) of HSV-1, and the virus titer was detected by plaque test after 4 days.

The detection results of HSV-1 replication in mouse brain are shown in FIG. 7.

The results showed that the virus titer in the brain of hnRNP-A2B1 knockout mice was significantly higher than that of wild-type mice (***, *P*<0.001).

The results showed that hnRNPA2B1 knockout promoted the replication of HSV-1 virus *in vivo,* indicating that hnRNPA2B1 may play an important role in anti-infection.

### Example 6: Knockout of hnRNPA2B improves the mortality caused by viral infection.

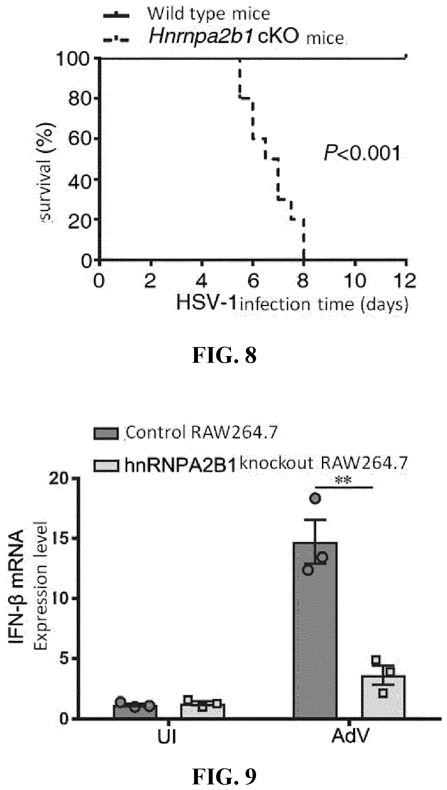

hnRNPA2B1 myeloid cell conditional knockout mice were constructed (as Example 3). Wild type mice and myeloid cell specific hnRNPA2B1 gene knockout (*Hnrnpa2b1* cKO) mice were intraperitoneally infected with 7×10⁸ plaque forming units (PFU) of HSV-1, and the death of mice was continuously observed. The results are shown in FIG. 8.

The results showed that the survival of hnRNP-A2B1 knockout mice decreased significantly (***, *P*<0.001).

The results showed that hnRNPA2B1 knockout can increase the mortality caused by viral infection, indicating that hnRNPA2B1 may play an important role in anti-infection.

### Example 7: Knockout of hnRNPA2B inhibits type I interferon induced by adenovirus (ADV) infection.

The second exon of hnRNPA2B1 gene was deleted by CRISPR/Cas9 technology to construct hnRNPA2B1 knockout RAW264.7 cell lines. The control RAW264.7 cells (ATCC) and hnRNPA2B1 knockout RAW264.7 cells were infected with AdV (MOI, 10; Ad5 type, Hanheng Biotechnology (Shanghai) Co., Ltd.), the adherent cells were collected 7 hours after infection, the total RNA of the cells was extracted and IFN-β mRNA expression level was detected by real-time fluorescence quantitative PCR.

The IFN-β mRNA expression level is shown in FIG. 9.

The results showed that hnRNPA2B1 knockout can significantly inhibit IFN-β production induced by AdV infection.

The results showed that hnRNPA2B1 knockout resulted in the decrease of type I interferon induced by AdV infection, indicating that hnRNPA2B1 may play an important role in anti-infection.

All documents mentioned in the present disclosure are cited as references in the present application, just as each document is cited as a reference separately.

## Claims

1. Heterogeneous nuclear ribonucleoprotein A2B 1 (hnRNPA2B 1), a nucleic acid molecule encoding the protein, a promoter thereof for use in the prevention and/or treatment of an infectious disease caused by DNA virus and/or an infection associated disease and/or symptom caused by DNA virus,
wherein the hnRNPA2B 1 is selected from:
(a) a polypeptide having the amino acid sequence set forth in SEQ ID NO: 2 or SEQ ID NO: 4; or
(b) a protein or polypeptide that has a sequence identity of more than 80%, such as 80%, 85%, 90%, 95%, 98%, 99% with the amino acid sequence set forth in SEQ ID NO: 2 or SEQ ID NO: 4, and has an infection inhibitory activity; or
wherein the nucleic acid is selected from:
(d) a nucleic acid molecule having the nucleotide sequence set forth in SEQ ID NO: 1 or SEQ ID NO: 3; or
(e) a molecule that hybridizes with the nucleotide sequence defined in (d) under a strict condition, wherein the strict condition is selected from a condition in that: (1) hybridization and elution at 0.2×SSC, 0. I%SDS, 60°C; or (2) addition of denaturant during hybridization, wherein the denaturant comprises 50% (v/v) formamide, 0.1% fetal bovine serum/0.1% Ficoll, at 42 °C; or (3) hybridization that occurs only when the identity between the two sequences is more than 80%, more than 85% or more than 90%, more preferably more than 95%; or
(f) a nucleic acid molecule, which has a sequence identity of more than 80% , such as 80%, 85%, 90%, 95%, 98%, 99% with the nucleotide sequences set forth in SEQ ID NO: 1 or SEQ ID NO:3, and encodes a protein or peptide that is active in preventing and/or treating an infectious disease and/or an infection associated disease and/or symptom; or
(g) the promoter is selected from: agents that increase the protein level of hnRNPA2B1 or promote the function of hnRNPA2B1, the agents being selected from an overexpression vector of hnRNPA2B 1 or hnRNPA2B 1 coding sequence; exogenous hnRNPA2B 1; a naked DNA of hnRNPA2B 1 coding sequence; a liposome encapsulated DNA of hnRNPA2B 1 coding sequence; hnRNPA2B 1 precursor protein or conjugate or complex that can be transformed into hnRNPA2B1 *in vivo;*
wherein the hnRNPA2B1, the nucleic acid molecule or the promoter is active in preventing and/or treating an infectious disease caused by DNA virus and/or an infection associated disease and/or symptom caused by DNA virus.

2. The hnRNPA2B 1, nucleic acid molecule, or promoter for use according to claim 1, wherein the infection is an infection caused by one or more viruses selected from: herpes simplex virus, hepatitis B virus, adenovirus, poxvirus, small DNA virus and adeno-associated virus.

3. The hnRNPA2B 1, nucleic acid molecule, or promoter for use according to claim 1, wherein the infection associated disease and/or symptom caused by a DNA virus is one or more selected from the group consisting of: pathological injury caused by infection; insufficient or excessive production of cytokines such as interferon after infection; endotoxic shock; inflammatory injury of organs; multiple organ failure, for example, the organ is selected from: liver, spleen, brain, kidney, heart, lung, stomach and intestine; chronic inflammatory diseases caused by viral infections, such as autoimmune diseases.

4. The hnRNPA2B 1, nucleic acid molecule, or promoter for use according to claim 1, wherein the product is a pharmaceutical composition or kit, for example, the form of which is suitable for an administration selected from the group consisting of oral administration, injection, such as direct naked DNA or protein injection, liposome encapsulated DNA or protein injection, gold coated gene gun bombardment, reproduction defective bacteria carrying plasmid DNA, replication deficient adenovirus carrying a protein encoded by a target DNA method or target gene, electroporation, nasal administration, pulmonary administration, oral administration, transdermal administration and intratumoral administration.

5. The hnRNPA2B 1, nucleic acid molecule, or promoter for use according to claim 1, wherein the product also contains other agents for prevention and/or treatment of an infectious disease caused by a DNA virus and/or an infection associated disease and/or symptom caused by a DNA virus, which is one or more selected from clinically common antibiotics (including β-lactams (penicillins and cephalosporins), aminoglycosides, tetracyclines, chloramphenicols, macrolides, antifungal antibiotics and anti-tuberculosis antibiotics); clinically common antiviral drugs (tricyclic amines, pyrophosphates, protease inhibitors, nucleoside drugs, interferon, antisense oligonucleotides, etc.); clinically common immunosuppressants (including glucocorticoid, cyclophosphamide, chloroquine, cyclosporine A, Tripterygium wilfordii, traditional Chinese medicine preparation and anti-TNF monoclonal antibody).

6. A pharmaceutical composition or kit for use in the prevention and/or treatment of an infectious disease caused by a DNA virus and/or an infection associated disease and/or symptom caused by a DNA virus, which comprises:
(A) a therapeutically or prophylactically effective amount of hnRNPA2B 1, a nucleic acid molecule encoding the protein, a promoter thereof wherein the hnRNPA2B 1 is selected from:
(a) a polypeptide having the amino acid sequence set forth in SEQ ID NO: 2 or SEQ ID NO: 4; or
(b) a protein or polypeptide that has a sequence identity of more than 80%, such as 80%, 85%, 90%, 95%, 98%, 99% with the amino acid sequence set forth in SEQ ID NO: 2 or SEQ ID NO: 4, and has an infection inhibitory activity; or
wherein the nucleic acid is selected from:
(d) a nucleic acid molecule having the nucleotide sequence set forth in SEQ ID NO: 1 or SEQ ID NO: 3; or
(e) a molecule that hybridizes with the nucleotide sequence defined in (d) under a strict condition, wherein the strict condition is selected from a condition in that: (1) hybridization and elution at 0.2×SSC, 0.1%SDS, 60°C; or (2) addition of denaturant during hybridization, wherein the denaturant comprises 50% (v/v) formamide, 0.1% fetal bovine serum/0.1% Ficoll, at 42 °C; or (3) hybridization that occurs only when the identity between the two sequences is more than 80%, more than 85% or more than 90%, more preferably more than 95%; or
(f) a nucleic acid molecule, which has a sequence identity of more than 80%, such as 80%, 85%, 90%, 95%, 98%, 99% with the nucleotide sequences set forth in SEQ ID NO: 1 or SEQ ID NO:3, and encodes a protein or peptide that is active in preventing and/or treating an infectious disease and/or an infection associated disease and/or symptom; or
(g) the promoter is selected from: agents that increase the protein level of hnRNPA2B 1 or promote the function of hnRNPA2B 1, the agent being selected from an overexpression vector of hnRNPA2B 1 or hnRNPA2B 1 coding sequence; exogenous hnRNPA2B 1; a naked DNA of hnRNPA2B 1 coding sequence; a liposome encapsulated DNA of hnRNPA2B 1 coding sequence; hnRNPA2B1 precursor protein or conjugate or complex that can be transformed into hnRNPA2B1 *in vivo;*
wherein the hnRNPA2B1, the nucleic acid molecule or the promoter is active in preventing and/or treating an infectious disease caused by DNA virus and/or an infection associated disease and/or symptom caused by DNA virus; and
(B) a pharmaceutically or immunologically acceptable carrier or excipient.

7. The pharmaceutical composition or kit for use according to claim 6, wherein the pharmaceutical composion or kit further comprises one or more other actives for preventing or treating an infectious disease caused by a DNA virus and its associated disease and/or symptom caused by a DNA virus, which is one or more selected from clinically common antibiotics (including β-lactams (penicillins and cephalosporins), aminoglycosides, tetracyclines, chloramphenicols, macrolides, antifungal antibiotics and anti-tuberculosis antibiotics); clinically common antiviral drugs (tricyclic amines, pyrophosphates, protease inhibitors, nucleoside drugs, interferon, antisense oligonucleotides, etc.); clinically common immunosuppressants (including glucocorticoid, cyclophosphamide, chloroquine, cyclosporine A, Tripterygium wilfordii, traditional Chinese medicine preparation and anti-TNF monoclonal antibody).

## Patentansprüche

1. Heterogenes nukleäres Ribonukleoprotein A2B1 (hnRNPA2B1), das Protein codierendes Nukleinsäuremolekül, Promotor davon, zur Verwendung bei der Vorbeugung und/oder Behandlung einer durch DNA-Virus verursachten Infektionskrankheit und/oder einer infektionsassoziierten Krankheit und/oder eines infektionsassoziierten Symptoms, die bzw. das durch DNA-Virus verursacht wird,
wobei das hnRNPA2B1 ausgewählt ist aus:
(a) einem Polypeptid mit der Aminosäuresequenz gemäß SEQ ID NO: 2 oder SEQ ID NO: 4; oder
(b) einem Protein oder Polypeptid, das eine Sequenzidentität von mehr als 80 %, wie 80 %, 85 %, 90 %, 95 %, 98 %, 99 %, mit der Aminosäuresequenz gemäß SEQ ID NO: 2 oder SEQ ID NO: 4 und eine infektionshemmende Aktivität aufweist; oder
wobei die Nukleinsäure ausgewählt ist aus:
(d) einem Nukleinsäuremolekül mit der Nukleotidsequenz gemäß SEQ ID NO: 1 oder SEQ ID NO: 3; oder
(e) einem Molekül, das mit der unter (d) definierten Nukleotidsequenz unter einer strikten Bedingung hybridisiert, wobei die strikte Bedingung aus einer Bedingung ausgewählt ist, bei der Folgendes gilt: (1) Hybridisierung und Elution bei 0,2×SSC, 0,1% SDS, 60 °C; oder (2) Zugabe eines Denaturierungsmittels während der Hybridisierung, wobei das Denaturierungsmittel 50 Vol.-% Formamid, 0,1 % fötales Rinderserum/0,1 % Ficoll umfasst, bei 42 °C; oder (3) Hybridisierung, die nur stattfindet, wenn die Identität zwischen den beiden Sequenzen größer 80 %, größer 85 % oder größer 90 %, bevorzugter größer 95 % ist; oder
(f) einem Nukleinsäuremolekül, das eine Sequenzidentität von mehr als 80 %, wie 80 %, 85 %, 90 %, 95 %, 98 %, 99 %, mit den Nukleotidsequenzen gemäß SEQ ID NO:1 oder SEQ ID NO:3 aufweist und ein Protein oder Peptid codiert, das beim Vorbeugen und/oder Behandeln einer Infektionskrankheit und/oder einer infektionsassoziierten Krankheit und/oder eines infektionsassoziierten Symptoms wirkt; oder
(g) der Promotor ausgewählt ist aus: Agentien, die den Proteinspiegel von hnRNPA2Bl erhöhen oder die Funktion von hnRNPA2Bl fördern, wobei die Agentien aus einem Überexpressionsvektor von hnRNPA2B1 oder hnRNP A2B 1-Codiersequenz; exogenem hnRNPA2B1; einer nackten DNA der hnRNPA2B1-Codiersequenz; einer liposomenverkapselten DNA der hnRNPA2B1-Codiersequenz; hnRNPA2B1-Vorläuferprotein oder -Konjugate oder -Komplex, das bzw. der *in vivo* in hnRNPA2B1 überführen lässt;
wobei das hnRNPA2B 1, das Nukleinsäuremolekül bzw. der Promotor beim Vorbeugen und/oder Behandeln einer durch DNA-Virus verursachten Infektionskrankheit und/oder einer infektionsassoziierten Krankheit und/oder eines infektionsassoziierten Symptoms, die bzw. das durch DNA-Virus verursacht wird, wirkt.

2. hnRNPA2B 1, Nukleinsäuremolekül oder Promotor zur Verwendung nach Anspruch 1, wobei es sich bei der Infektion um eine durch ein oder mehrere Viren, die aus Herpes-simplex-Virus, Hepatitis-B-Virus, Adenovirus, Poxvirus, kleinem DNA-Virus und adeno-assoziiertem Virus ausgewählt sind, verursachte Infektion handelt.

3. hnRNPA2B 1, Nukleinsäuremolekül oder Promotor zur Verwendung nach Anspruch 1, wobei es sich bei der infektionsassoziierten Krankheit und/oder des infektionsassoziierten Symptoms, die bzw. das durch ein DNA-Virus verursacht wird, um eine/ein oder mehrere aus der Gruppe bestehend aus pathologischer Verletzung, die durch eine Infektion verursacht wird, unzureichender oder übermäßiger Produktion von Cytokinen wie Interferon nach einer Infektion, endotoxischem Schock, entzündlicher Verletzung von Organen, Multiorganversagen, wobei das Organ beispielsweise ausgewählt ist aus Leber, Milz, Gehirn, Niere, Herz, Lunge, Magen und Darm, chronischen Entzündungskrankheiten, die durch Virusinfektionen verursacht werden, wie z.B. Autoimmunerkrankungen, ausgewählte Krankheiten bzw. Symptome handelt.

4. hnRNPA2B 1, Nukleinsäuremolekül oder Promotor zur Verwendung nach Anspruch 1, wobei es sich bei dem Produkt um eine pharmazeutische Zusammensetzung bzw. ein Kit handelt, deren bzw. dessen Form für eine Verabreichung geeignet ist, die aus der Gruppe bestehend aus oraler Verabreichung, Injektion wie direkter Injektion von nackter DNA bzw. nacktem Protein, Injektion von liposomenverkapselter DNA bzw. liposomenverkapseltem Protein, Beschuss mit der Genkanone mit Goldbeschichtung, reproduktionsdefekten Bakterien, die Plasmid-DNA tragen, replikationsdefizientem Adenovirus, das ein durch eine Ziel-DNA-Methode oder ein Zielgen codiertes Protein trägt, Elektroporation, nasaler Verabreichung, pulmonaler Verabreichung, oraler Verabreichung, transdermaler Verabreichung und intratumoraler Verabreichung ausgewählt ist.

5. hnRNPA2B 1, Nukleinsäuremolekül oder Promotor zur Verwendung nach Anspruch 1, wobei das Produkt auch andere Agentien zur Vorbeugung und/oder Behandlung einer durch ein DNA-Virus verursachten Infektionskrankheit und/oder einer infektionsassoziierten Krankheit und/oder eines infektionsassoziierten Symptoms, die bzw. das durch ein DNA-Virus verursacht wird, enthält, bei denen es sich um ein oder mehrere handelt, die aus klinisch üblichen Antibiotika (einschließlich β-Lactamen (Penicilline und Cephalosporine), Aminoglykosiden, Tetracyclinen, Chloramphenicolen, Makroliden, antimykotischen Antibiotika und Anti-Tuberkulose-Antibiotika), klinisch üblichen antiviralen Arzneistoffen (trizyklische Amine, Pyrophosphate, Proteasehemmer, Nukleosidarzneistoffe, Interferon, Antisense-Oligonukleotide usw.), klinisch üblichen Immunsuppressiva (einschließlich Glucocorticoid, Cyclophosphamid, Chloroquin, Cyclosporin A, Tripterygium wilfordii, Präparaten der traditionellen chinesischen Medizin und monoklonalem Anti-TNF-Antikörper) ausgewählt sind.

6. Pharmazeutische Zusammensetzung oder Kit zur Verwendung bei der Vorbeugung und/oder Behandlung einer durch ein DNA-Virus verursachten Infektionskrankheit und/oder einer infektionsassoziierten Krankheit und/oder eines infektionsassoziierten Symptoms, die bzw. das durch ein DNA-Virus verursacht wird, die bzw. das Folgendes umfasst:
(A) eine therapeutisch oder prophylaktisch wirksame Menge von hnRNPA2B1, einem das Protein codierenden Nukleinsäuremolekül, einem Promotor davon, wobei das hnRNPA2B 1 ausgewählt ist aus:
(a) einem Polypeptid mit der Aminosäuresequenz gemäß SEQ ID NO: 2 oder SEQ ID NO: 4; oder
(b) einem Protein oder Polypeptid, das eine Sequenzidentität von mehr als 80 %, wie 80 %, 85 %, 90 %, 95 %, 98 %, 99 %, mit der Aminosäuresequenz gemäß SEQ ID NO: 2 oder SEQ ID NO: 4 und eine infektionshemmende Aktivität aufweist; oder
wobei die Nukleinsäure ausgewählt ist aus:
(d) einem Nukleinsäuremolekül mit der Nukleotidsequenz gemäß SEQ ID NO: 1 oder SEQ ID NO: 3; oder
(e) einem Molekül, das mit der unter (d) definierten Nukleotidsequenz unter einer strikten Bedingung hybridisiert, wobei die strikte Bedingung aus einer Bedingung ausgewählt ist, bei der Folgendes gilt:
(1) Hybridisierung und Elution bei 0,2×SSC, 0,1% SDS, 60 °C; oder (2) Zugabe eines Denaturierungsmittels während der Hybridisierung, wobei das Denaturierungsmittel 50 Vol.-% Formamid, 0,1 % fötales Rinderserum/0,1 % Ficoll umfasst, bei 42 °C; oder (3) Hybridisierung, die nur stattfindet, wenn die Identität zwischen den beiden Sequenzen größer 80 %, größer 85 % oder größer 90 %, bevorzugter größer 95 % ist; oder
(f) einem Nukleinsäuremolekül, das eine Sequenzidentität von mehr als 80 %, wie 80 %, 85 %, 90 %, 95 %, 98 %, 99 %, mit den Nukleotidsequenzen gemäß SEQ ID NO:1 oder SEQ ID NO:3 aufweist und ein Protein oder Peptid codiert, das beim Vorbeugen und/oder Behandeln einer Infektionskrankheit und/oder einer infektionsassoziierten Krankheit und/oder eines infektionsassoziierten Symptoms wirkt; oder
(g) der Promotor ausgewählt ist aus: Agentien, die den Proteinspiegel von hnRNPA2Bl erhöhen oder die Funktion von hnRNPA2Bl fördern, wobei die Agentien aus einem Überexpressionsvektor von hnRNPA2B1 oder hnRNPA2B1-Codiersequenz; exogenem hnRNPA2B1; einer nackten DNA der hnRNPA2B1-Codiersequenz; einer liposomenverkapselten DNA der hnRNPA2B1-Codiersequenz; hnRNPA2B1-Vorläuferprotein oder - Konjugate oder -Komplex, das bzw. der *in vivo* in hnRNPA2B1 überführen lässt;
wobei das hnRNPA2B1, das Nukleinsäuremolekül bzw. der Promotor beim Vorbeugen und/oder Behandeln einer durch DNA-Virus verursachten Infektionskrankheit und/oder einer infektionsassoziierten Krankheit und/oder eines infektionsassoziierten Symptoms, die bzw. das durch DNA-Virus verursacht wird, wirkt; und
(B) einen pharmazeutisch oder immunologisch unbedenklichen Träger oder Exzipienten.

7. Pharmazeutische Zusammensetzung oder Kit zur Verwendung nach Anspruch 6, wobei die pharmazeutische Zusammensetzung bzw. das Kit ferner einen oder mehrere Wirkstoffe zum Vorbeugen und/oder Behandeln einer durch ein DNA-Virus verursachten Infektionskrankheit und ihrer assoziierten Krankheit und/oder ihres assoziierten Symptoms, die bzw. das durch ein DNA-Virus verursacht wird, umfasst, bei denen es sich um einen oder mehrere handelt, die aus klinisch üblichen Antibiotika (einschließlich β-Lactamen (Penicilline und Cephalosporine), Aminoglykosiden, Tetracyclinen, Chloramphenicolen, Makroliden, antimykotischen Antibiotika und Anti-Tuberkulose-Antibiotika), klinisch üblichen antiviralen Arzneistoffen (trizyklische Amine, Pyrophosphate, Proteasehemmer, Nukleosidarzneistoffe, Interferon, Antisense-Oligonukleotide usw.), klinisch üblichen Immunsuppressiva (einschließlich Glucocorticoid, Cyclophosphamid, Chloroquin, Cyclosporin A, Tripterygium wilfordii, Präparaten der traditionellen chinesischen Medizin und monoklonalem Anti-TNF-Antikörper) ausgewählt sind.

## Revendications

1. Ribonucléoprotéine nucléaire hétérogène A2B1 (hnRNP A2B 1), molécule d'acide nucléique codant pour la protéine, promoteur correspondant pour une utilisation dans la prévention et/ou le traitement d'une maladie infectieuse causée par un virus à ADN et/ou d'une maladie et/ou d'un symptôme associé(e) à une infection causé(e) par un virus à ADN,
la hnRNPA2B1 étant choisie parmi :
(a) un polypeptide ayant la séquence d'acides aminés indiquée dans la SEQ ID NO: 2 ou la SEQ ID NO: 4 ; ou
(b) une protéine ou un polypeptide qui possède une identité de séquence supérieure à 80 %, telle que de 80 %, 85 %, 90 %, 95 %, 98 %, 99 % avec la séquence d'acides aminés indiquée dans la SEQ ID NO: 2 ou la SEQ ID NO: 4, et possède une activité inhibitrice d'infection ; ou
l'acide nucléique étant choisi parmi :
(d) une molécule d'acide nucléique ayant la séquence de nucléotides indiquée dans la SEQ ID NO: 1 ou la SEQ ID NO: 3 ; ou
(e) une molécule qui s'hybride à la séquence de nucléotides définie en (d) sous une condition stricte, la condition stricte étant choisie parmi une condition dans laquelle : (1) hybridation et élution à 0,2 × SSC, 0,1 % SDS, 60 °C ; ou (2) ajout d'un dénaturant pendant l'hybridation, le dénaturant comprenant 50 % (v/v) de formamide, 0,1 % de sérum bovin fœtal/0,1 % Ficoll, à 42 °C ; ou (3) hybridation qui a lieu seulement lorsque l'identité entre les deux séquences est supérieure à 80 %, supérieure à 85 % ou supérieure à 90 %, plus préférablement supérieure à 95 %;
ou
(f) une molécule d'acide nucléique, qui possède une identité de séquence supérieure à 80 %, telle que de 80 %, 85 %, 90 %, 95 %, 98 %, 99 % avec les séquences de nucléotides indiquées dans la SEQ ID NO: 1 ou la SEQ ID NO: 3, et qui code pour une protéine ou un peptide qui est active/actif dans la prévention et/ou le traitement d'une maladie infectieuse et/ou d'une maladie et/ou d'un symptôme associé(e) à une infection ; ou
(g) le promoteur étant choisi parmi : des agents qui augmentent le taux de protéines de hnRNPA2B1 ou favorisent la fonction de hnRNPA2B1, les agents étant choisis parmi un vecteur de surexpression de hnRNP A2B 1 ou une séquence codant pour hnRNPA2B1 ; hnRNPA2B1 exogène ; un ADN nu de séquence codant pour hnRNPA2B1 ; un ADN, encapsulé dans un liposome, de séquence codant pour hnRNPA2B1 ; une protéine ou un conjugué ou un complexe précurseur de hnRNPA2B1 qui peut être transformé(e) en hnRNPA2B1 *in vivo* ;
la hnRNPA2B1, la molécule d'acide nucléique ou le promoteur étant active/actif dans la prévention et/ou le traitement d'une maladie infectieuse causée par un virus à ADN et/ou d'une maladie et/ou d'un symptôme associé(e) à une infection causé(e) par un virus à ADN.

2. hnRNPA2B1, molécule d'acide nucléique ou promoteur pour une utilisation selon la revendication 1, l'infection étant une infection causée par un ou plusieurs virus choisis parmi : virus de l'herpès simplex, virus de l'hépatite B, adénovirus, poxvirus, virus à petit ADN et virus adéno-associé.

3. hnRNPA2B1, molécule d'acide nucléique ou promoteur pour une utilisation selon la revendication 1, la maladie et/ou le symptôme associé(e) à une infection, causé(e) par un virus à ADN étant l'un ou plusieurs choisis dans le groupe constitué par : lésion pathologique causée par une infection ; production insuffisante ou excessive de cytokines telles que l'interféron après une infection ; choc endotoxique ; lésion inflammatoire d'organes ; défaillance de plusieurs organes, par exemple, l'organe étant choisi parmi : le foie, la rate, le cerveau, les reins, le coeur, les poumons, l'estomac et l'intestin ; maladies inflammatoires chroniques causées par des infections virales, telles que des maladies auto-immunes.

4. hnRNPA2B1, molécule d'acide nucléique ou promoteur pour une utilisation selon la revendication 1, le produit étant une composition ou un kit pharmaceutique, par exemple, la forme de laquelle/duquel étant appropriée pour une administration choisie dans le groupe constitué par une administration orale, une injection, telle qu'une injection directe d'ADN nu ou de protéine, une injection d'ADN ou de protéine encapsulé(e) dans un liposome, un bombardement par un pistolet à gènes revêtus d'or, des bactéries défectueuses pour la reproduction transportant de l'ADN plasmidique, un adénovirus déficient en réplication transportant une protéine codée par un procédé d'ADN cible ou un gène cible, une électroporation, une administration nasale, une administration pulmonaire, une administration orale, une administration transdermique et une administration intratumorale.

5. hnRNPA2B1, molécule d'acide nucléique ou promoteur pour une utilisation selon la revendication 1, le produit contenant également d'autres agents pour la prévention et/ou le traitement d'une maladie infectieuse causée par un virus à ADN et/ou d'une maladie et/ou d'un symptôme associé(e) à une infection causé(e) par un virus à ADN, qui est l'un ou plusieurs choisis parmi les antibiotiques cliniquement courants (y compris les β-lactames (pénicillines et céphalosporines), les aminoglycosides, les tétracyclines, les chloramphénicols, les macrolides, les antibiotiques antifongiques et les antibiotiques antituberculeux)) ; les médicaments antiviraux cliniquement courants (amines tricycliques, pyrophosphates, inhibiteurs de protéase, médicaments nucléosidiques, interféron, oligonucléotides antisens, etc.); les immunosuppresseurs cliniquement courants (y compris les glucocorticoïdes, le cyclophosphamide, la chloroquine, la cyclosporine A, Tripterygium wilfordii, une préparation de médecine traditionnelle chinoise et un anticorps monoclonal anti-TNF).

6. Composition ou kit pharmaceutique pour une utilisation dans la prévention et/ou le traitement d'une maladie infectieuse causée par un virus à ADN et/ou d'une maladie et/ou d'un symptôme associé(e) à une infection causé(e) par un virus à ADN, qui comprend :
(A) une quantité thérapeutiquement ou prophylactiquement efficace de hnRNPA2B1, d'une molécule d'acide nucléique codant pour la protéine, d'un promoteur correspondant, la hnRNP A2B 1 étant choisie parmi :
(a) un polypeptide ayant la séquence d'acides aminés indiquée dans la SEQ ID NO: 2 ou la SEQ ID NO: 4 ; ou
(b) une protéine ou un polypeptide qui possède une identité de séquence supérieure à 80 %, telle que de 80 %, 85 %, 90 %, 95 %, 98 %, 99 % avec la séquence d'acides aminés indiquée dans la SEQ ID NO: 2 ou la SEQ ID NO: 4, et possède une activité inhibitrice d'infection ; ou
l'acide nucléique étant choisi parmi :
(d) une molécule d'acide nucléique ayant la séquence de nucléotides indiquée dans la SEQ ID NO: 1 ou la SEQ ID NO: 3 ; ou
(e) une molécule qui s'hybride à la séquence de nucléotides définie en (d) sous une condition stricte, la condition stricte étant choisie parmi une condition dans laquelle : (1) hybridation et élution à 0,2 × SSC, 0,1 % SDS, 60 °C ; ou (2) ajout d'un dénaturant pendant l'hybridation, le dénaturant comprenant 50 % (v/v) de formamide, 0,1 % de sérum bovin fœtal/0,1 % Ficoll, à 42 °C ; ou (3) hybridation qui a lieu seulement lorsque l'identité entre les deux séquences est supérieure à 80 %, supérieure à 85 % ou supérieure à 90 %, plus préférablement supérieure à 95 % ; ou
(f) une molécule d'acide nucléique, qui possède une identité de séquence supérieure à 80 %, telle que de 80 %, 85 %, 90 %, 95 %, 98 %, 99 % avec les séquences de nucléotides indiquées dans la SEQ ID NO: 1 ou la SEQ ID NO: 3, et qui code pour une protéine ou un peptide qui est active/actif dans la prévention et/ou le traitement d'une maladie infectieuse et/ou d'une maladie et/ou d'un symptôme associé(e) à une infection ; ou
(g) le promoteur étant choisi parmi : des agents qui augmentent le taux de protéines de hnRNP A2B 1 ou favorisent la fonction de hnRNPA2B1, les agents étant choisis parmi un vecteur de surexpression de hnRNPA2B1 ou une séquence codant pour hnRNP A2B 1 ; hnRNP A2B 1 exogène ; un ADN nu de séquence codant pour hnRNPA2B1 ; un ADN, encapsulé dans un liposome, de séquence codant pour hnRNPA2B1 ; une protéine ou un conjugué ou un complexe précurseur de hnRNPA2B1 qui peut être transformé(e) en hnRNPA2B1 *in vivo* ;
la hnRNPA2B1; la molécule d'acide nucléique ou le promoteur étant active/actif dans la prévention et/ou le traitement d'une maladie infectieuse causée par un virus à ADN et/ou d'une maladie et/ou d'un symptôme associé(e) à une infection causé(e) par un virus à ADN ; et
(B) un support ou un excipient pharmaceutiquement ou immunologiquement acceptable.

7. Composition ou kit pharmaceutique pour une utilisation selon la revendication 6, la composition ou le kit pharmaceutique comprenant en outre un ou plusieurs autres agents actifs pour la prévention ou le traitement d'une maladie infectieuse causée par un virus à ADN et sa maladie et/ou son symptôme associé(e) causé(e) par un virus à ADN, qui est l'un ou plusieurs choisis parmi les antibiotiques cliniquement courants (y compris les β-lactames (pénicillines et céphalosporines), les aminoglycosides, les tétracyclines, les chloramphénicols, les macrolides, les antibiotiques antifongiques et les antibiotiques antituberculeux)) ; les médicaments antiviraux cliniquement courants (amines tricycliques, pyrophosphates, inhibiteurs de protéase, médicaments nucléosidiques, interféron, oligonucléotides antisens, etc.);
les immunosuppresseurs cliniquement courants (y compris les glucocorticoïdes, le cyclophosphamide, la chloroquine, la cyclosporine A, Tripterygium wilfordii, une préparation de médecine traditionnelle chinoise et un anticorps monoclonal anti-TNF).
